# EUROPEAN PATENT SPECIFICATION

(11) **EP 2 330 186 B1**
(45) Date of publication and mention of the grant of the patent: **31.12.2014**
(21) Application number: 09814823.2
(22) Date of filing: 22.09.2009
(51) Int. Cl.: C12N 1/21, C12N 15/67, C07K 14/435

(54) **METHOD FOR SYNTHESIZING PROTEIN CONTAINING HIGH CONTENT OF SPECIFIC AMINO ACID THROUGH SIMULTANEOUS EXPRESSION WITH TRNA OF THE SPECIFIC AMINO ACID**
VERFAHREN ZUR SYNTHETISIERUNG EINES PROTEINS MIT HOHEM ANTEIL EINER SPEZIFISCHEN AMINOSÄURE DURCH SIMULTANE EXPRESSION MIT DER TRNA DER SPEZIFISCHEN AMINOSÄURE
MÉTHODE DE SYNTHÈSE D'UNE PROTÉINE À TENEUR ÉLEVÉE EN UN ACIDE AMINÉ SPÉCIFIQUE PAR EXPRESSION SIMULTANÉE AVEC UN ARN DE TRANSFERT (ARNT) DE L'ACIDE AMINÉ SPÉCIFIQUE

(30) Priority: 22.09.2008 KR 20080092906
(43) Date of publication of application: 08.06.2011
(73) Proprietor: Korea Advanced Institute of Science and Technology, Daejeon, 305-701 (KR)
(72) Inventor: LEE, Sang Yup, Daejeon 305-761 (KR); XIA, Xiaoxia, Daejeon 305-701 (KR); QIAN, Zhi Gang, Daejeon 305-701 (KR); BAEK, Jong Hwan, Daejeon 305-701 (KR)
(74) Representative: Hiebl, Inge Elisabeth
(86) International application number: PCT/KR2009/005394
(87) International publication number: WO 2010/033007

(56) References cited:
- WO-A2-2007/124493
- JP-A- 2005 046 068
- KR-B1- 100 489 500
- US-B1- 6 270 988
- US-B2- 6 821 755
- IMAMURA H ET AL: "High level expression of Thermococcus litoralis 4-alpha-glucanotransferase in a soluble form in Escherichia coli with a novel expression system involving minor arginine tRNAs and GroELS", FEBS LETTERS, ELSEVIER, AMSTERDAM, NL, vol. 457, no. 3, 3 September 1999 (1999-09-03), pages 393-396, XP004260188, ISSN: 0014-5793, DOI: 10.1016/S0014-5793(99)01081-9
- DRABKIN ET AL: "Preferential use in collagen synthesis of the same glycyl-tRNA species that is elevated in collagen-synthesizing tissues.", JOURNAL OF BIOLOGICAL CHEMISTRY, vol. 253, no. 17, 1 September 1978 (1978-09-01), pages 6233-6241, XP55033053, ISSN: 0021-9258
- CARPOUSIS A ET AL: "Preferential usage of tRNA isoaccepting species in collagen synthesis", JOURNAL OF BIOLOGICAL CHEMISTRY, THE AMERICAN SOCIETY OF BIOLOGICAL CHEMISTS, INC, US, vol. 252, no. 22, 25 November 1977 (1977-11-25), pages 8023-8026, XP008144476, ISSN: 0021-9258
- X.-X. XIA ET AL: "Native-sized recombinant spider silk protein produced in metabolically engineered Escherichia coli results in a strong fiber", PROCEEDINGS OF THE NATIONAL ACADEMY OF SCIENCES, vol. 107, no. 32, 10 August 2010 (2010-08-10), pages 14059-14063, XP55006380, ISSN: 0027-8424, DOI: 10.1073/pnas.1003366107
- AGAMEMNON CARPOUSIS ET AL.: 'Preferential Usage of tRNA Isoaccepting Species in Collagen Synthesis.' THE JOURNAL OF BIOLOGICAL CHEMISTRY vol. 252, no. 22, 25 November 1977, pages 8023 - 8026, XP008144476

### SEQUENCE LISTING

<110> Korea Advanced Institute of Science and Technology
<120> METHOD OF PREPARING PROTEIN HAVING HIGH CONTENT OF SPECIFIC AMINO ACID BY CO-EXPRESSION WITH TRNA OF SPECIFIC AMINO ACID
<130> 22563EP
<140> EP 09 814 823.2
   <141> 2009-09-22
<150> KR 10-2008-0092906
   <151> 2008-09-22
<160> 19
<170> PatentIn version 3.5
<210> 1
   <211> 31
   <212> DNA
   <213> Artificial Sequence
<220>
   <223> primer
<400> 1
   gctcgatatc taacgacgca gaaatgcgaa a 31
<210> 2
   <211> 33
   <212> DNA
   <213> Artificial Sequence
<220>
   <223> primer
<400> 2
   cattggatcc taagattaca gcctgaggct gtg 33
<210> 3
   <211> 35
   <212> DNA
   <213> Artificial Sequence
<220>
   <223> primer
<400> 3
   ggctcgcatg ctcatgtttg acagcttatc atcga 35
<210> 4
   <211> 40
   <212> DNA
   <213> Artificial Sequence
<220>
   <223> primer
<400> 4
   attgtcgact gctgcagtaa gattacagcc tgaggctgtg 40
<210> 5
   <211> 125
   <212> DNA
   <213> Artificial Sequence
<220>
   <223> primer
<400> 5
   tatggctagc ggtcagggtg gctacggtca gggcggtgca ggccaaggtg gtgcaggtgc 60
   tgcggcagct gctgcggcgg ctggcggtgc aggtcaaggt ggccagggtg gttacactag 120
   ttaac 125
<210> 6
   <211> 127
   <212> DNA
   <213> Artificial Sequence
<220>
   <223> primer
<400> 6
   tcgagttaac tagtgtaacc accctggcca ccttgacctg caccgccagc cgccgcagca 60
   gctgccgcag cacctgcacc accttggcct gcaccgccct gaccgtagcc accctgaccg 120
   ctagcca 127
<210> 7
   <211> 5
   <212> PRT
   <213> Artificial Sequence
<220>
   <223> repeating sequence of elastin
<400> 7
   Gly Val Gly Val Pro
   1 5
<210> 8
   <211> 4
   <212> PRT
   <213> Artificial Sequence
<220>
   <223> repeating sequence of elastin
<400> 8
   Val Pro Gly Gly
   1
<210> 9
   <211> 6
   <212> PRT
   <213> Artificial Sequence
<220>
   <223> repeating sequence of elastin
<400> 9
   Ala Pro Gly Val Gly Val
   1 5
<210> 10
   <211> 6
   <212> PRT
   <213> Artificial Sequence
<220>
   <223> repeating sequence of silk fibroin
<400> 10
   Gly Ala Gly Ala Gly Ser
   1 5
<210> 11
   <211> 5
   <212> PRT
   <213> Artificial Sequence
<220>
   <223> repeating sequence of byssus
<400> 11
   Gly Pro Gly Gly Gly
   1 5
<210> 12
   <211> 6
   <212> PRT
   <213> Artificial Sequence
<220>
   <223> repeating sequence of flagelliform silk
<400> 12
   Gly Pro Gly Gly Ala Ala
   1 5
<210> 13
   <211> 5
   <212> PRT
   <213> Artificial Sequence
<220>
   <223> repeating sequence of dragline silk
<400> 13
   Gly Pro Gly Gln Gln
   1 5
<210> 14
   <211> 5
   <212> PRT
   <213> Artificial Sequence
<220>
   <223> repeating sequence of dragline silk
<400> 14
   Gly Pro Gly Gly Tyr
   1               5
<210> 15
   <211> 7
   <212> PRT
   <213> Artificial Sequence
<220>
   <223> repeating sequence of dragline silk
<400> 15
   Gly Gly Tyr Gly Pro Gly Ser
   1               5
<210> 16
   <211> 15
   <212> PRT
   <213> Artificial Sequence
<220>
   <223> repeating sequence of collagen
<400> 16
   Gly Ala Pro Gly Ala Pro Gly Ser Gln Gly Ala Pro Gly Leu Gln
   1               5                   10                  15
<210> 17
   <211> 15
   <212> PRT
   <213> Artificial Sequence
<220>
   <223> repeating sequence of collagen
<400> 17
   Gly Ala Pro Gly Thr Pro Gly Pro Gln Gly Leu Pro Gly Ser Pro
   1               5                   10                  15
<210> 18
   <211> 13
   <212> PRT
   <213> Artificial Sequence
<220>
   <223> repeating sequence of keratin
<400> 18
   Ala Lys Leu Lys Leu Ala Glu Ala Lys Leu Glu Leu Ala
   1               5                   10
<210> 19
   <211> 38
   <212> PRT
   <213> Artificial Sequence
<220>
   <223> repeating sequence of sericin
<400> 19
   Ser Ser Thr Gly Ser Ser Ser Asn Thr Asp Ser Asn Ser Asn Ser Val
   1               5                   10                  15
   Gly Ser Ser Thr Ser Gly Gly Ser Ser Thr Tyr Gly Tyr Ser Ser Asn
               20                  25                  30
   Ser Arg Asp Gly Ser Val
           35

## Description

### TECHNICAL FIELD

The present invention relates to a method of increasing the expression of a target protein being silk protein by co-expression of a gene encoding the target protein having a high content of a specific amino acid being selected from the group consisting of glycine, alanine and serine with a nucleotide sequence encoding the tRNA of the specific amino acid.

### BACKGROUND ART

Repetitive protein polymers consisting of repeats of various natural or non-natural amino acids have received attention as important substances (Barron et al., Curr. Opin. Chem. Biol., 3:681, 1999; Huang et al., J. Macromolecular Science, Part C: Polymer Reviews, 47:29, 2007; Kluge et al., Trends in Biotechnology, 26 (5) :244, 2007; Nagarsekar et al., Drug Target, 7:11, 1999; Vendrely et al., Macromol. Biosci., 7:401, 2007). This is because the protein polymers are made based on genetic information (gene), and thus the various properties of the polymer proteins, such as length or steric properties, can be accurately controlled, unlike polymers made by a chemical synthesis method. Also, because the protein polymers can be prepared to have desired mechanical, chemical and biological properties, including biocompatibility and biodegradability, they can be advantageously used in the biomaterial engineering and tissue engineering fields. Repeating sequences of typical repeat proteins having the properties of protein polymers include elastin (GVGVP, VPGG, APGVGV), silk fibroin (GAGAGS), byssus (GPGGG), flagelliform silk (GPGGx), dragline silk (GPGQQ), GPGGY, GGYGPGS), collagen (GAPGAPGSQGAPGLQ, GAPGTPGPQGLPGSP), keratin (AKLKLAEAKLELA), sericin (SSTGSSSNTDSNSNSVGSSTSGGSSTYGYSSNSRDGSV), and synthetic repetitive protein (Kumar et al., Biomacromolecules, 7:2543, 2006) .

Meanwhile, systems of expressing recombinant repeat proteins in bacteria, including *E*. *coli,* yeasts such as *P*. *pastoris,* insect cells infected with baculovirus, transformed potato, tobacco, rat cells and the like, were developed. The size of proteins expressible in such systems was 3-163 kDa (Huang et al., J. Macromolecular Science, Part C: Polymer Reviews, 47:29, 2007; Scheller et al., Nat. Biotechnol., 19:573, 2001). Particularly, *E*. *coli* is a bacterium which has been studied, and it is easy to handle and can be industrially cultured at a relatively low cost, and thus can be advantageously used as a host for producing recombinant proteins. However, in the case of *E. coli,* because of the limitation of a translation apparatus, the yield of the production of repetitive proteins by high-concentration fermentation is as extremely low as about 140-360 mg/L depending on the size of the proteins (Fahnestock et al., Reviews in Molecular Biotechnology, 74:105, 1997; Scheibel et al., Microbial Cell Factories, 3, 2004). It has been difficult to produce repetitive proteins, particularly large amounts of high-molecular-weight repetitive proteins determining the mechanical properties of polymers, in *E*. *coli* (Fahnestock et al., Reviews in Molecular Biotechnology, 74:105, 2000; Huang et al., J. Biol. Chem., 278(46):46117, 2003; Lewis et al., Protein Expres. Purif., 7:400, 1996; Tsung et al., J. Biol. Chem., 264(8):4428, 1989).

The most typical method capable of improving the expression of a specific synthetic gene is to express the gene together with the tRNA of a codon which is essential for the synthesis of the protein but is rare in the host. Examples of this rare codon tRNA include *ile*X tRNA, *argU* tRNA, *thrU* tRNA, t*yr*U tRNA, *gly*T tRNA, *thr*T tRNA, *arg*W tRNA, *met*T tRNA, *leu*W tRNA, *pro*L tRNA, etc.

If codon usage requiring tRNA, which is less present or absent, for overexpression of a foreign gene in *E. coli,* has problems, translation cannot be accurately performed (Baca et al., Int. J. Parasitology, 30:113, 2000; Goldman et al., J. Mol. Biol., 245:467, 1995; Kane et al., Curr. Opin. Biotechnol., 6:494, 1995; Kurland et al., Curr. Opin. Biotechnol., 7: 489, 1996;). In order to prevent a translational delay or early translational termination, a translational frameshift, an incorrect amino acid linkage, etc., which can occur due to the lack of tRNA, studies focused on overexpressing a foreign protein together with a specific tRNA to solve the codon bias problems and increase the expression of the foreign protein have been reported (Blattner et al., WO 2007/124493; Catherine et al., WO 00/36123; Imamura et al., FEBS Letters, 457:393, 1999; Shin et al., Biotechnol. Bioprocess Eng., 6:301, 2001; Ulrich et al., US 6270988). In addition, in order to produce a protein comprising a non-natural amino acid, co-overexpression of the protein with a specific tRNA has been used (Anderson et al., US 2006/160175; Tirrell et al., US 2008/160609; Shigeyuki et al., EP 1911840).

Document DRABKIN et al.: "Preferential use in collagen synthesis of the same glycyl-tRNA species that is elevated in collagen-synthesizing tissues.", JOURNAL OF BIOLOGICAL CHEMISTRY, vol. 253, no. 17, September 1, 1978, pages 6233-6241 discloses the functional significance of differences in the tRNA populations between collagen- and non-collagen-synthesizing tissues in 17-day chick embryos.

Document CARPOUSIS A. et al.: "Preferential usage of tRNA isoaccepting species in collagen synthesis", JOURNAL OF BIOLOGICAL CHEMISTRY, THE AMERICAN SOCIETY OF BIOLOGICAL CHEMISTS, Inc, US, vol. 252, no. 22, November 25, 1977, pages 8023-8026 pages a new double-label technique for determining the usage of individual isoacception tRNA species by *in vitro* protein-synthesizing systems.

As described above, there has been an example of co-expressing a protein with rare tRNA to increase the production of the protein, but there has been no attempt to increase the expression of a specific repetitive protein by co-expressing the protein with tRNA of amino acid which is much present in the protein to be produced.

Accordingly, the present inventors have found that, if a target protein such as a repetitive protein is co-expressed with tRNA of amino acid which is abundantly present in the protein, the expression of the target protein will be significantly increased, thereby completing the present invention.

### DISCLOSURE OF INVENTION

It is an object of the present invention to provide a recombinant microorganism transformed with both a protein gene having a high content of a specific amino acid and a nucleotide sequence encoding the tRNA of the specific amino acid.

Another object of the present invention is to provide a method of preparing a protein having a high content of a specific amino acid by culturing said microorganism.

To achieve the above objects, the present invention provides a recombinant microorganism transformed with both a gene encoding a target protein having a specific amino acid content of 10% or more and a nucleotide sequence encoding the tRNA of the specific amino acid wherein the target protein is silk protein and the specific amino acid is selected from the group consisting of glycine, alanine and serine.

The present invention also provides a recombinant vector containing both a gene encoding a target protein having a specific amino acid content of 10% or more and a nucleotide sequence encoding the tRNA of the specific amino acid, wherein the target protein is silk protein and the specific amino acid is selected from the group consisting of glycine, alanine and serine.

The present invention also provides a method of preparing a protein having a specific amino acid content of 10% or more, characterized in that the method comprises culturing said recombinant microorganism according to claim 1 to express a target protein having a specific amino acid content of 10% or more, and collecting the expressed target protein, wherein the target protein is silk protein and the specific amino acid is selected from the group consisting of glycine, alanine and serine.

In the present invention, the target protein is silk protein.

Other features and embodiments of the present invention will be more apparent from the following detailed descriptions and the appended claims.

### BRIEF DESCRIPTION OF THE DRAWINGS

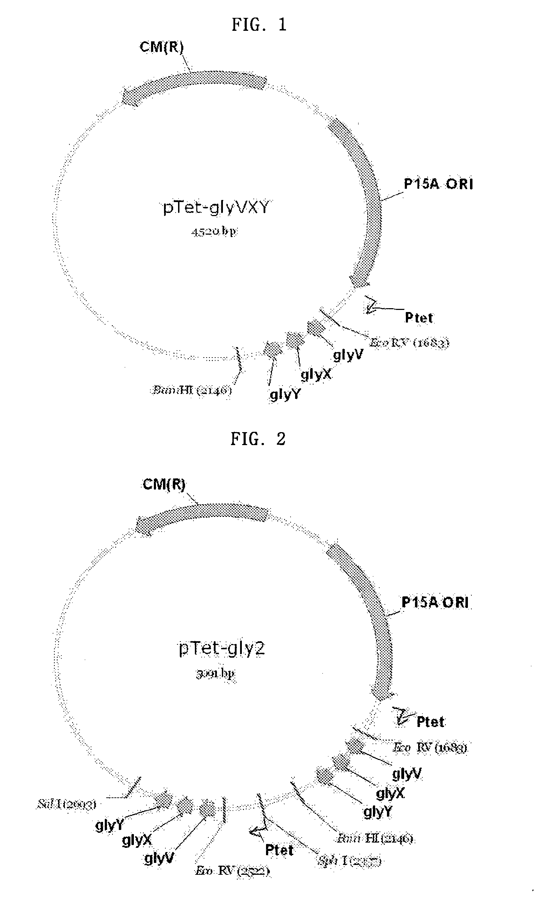

FIG. 1 is a gene map of the plasmid pTet-glyVXY.
FIG. 2 is a gene map of the plasmid pTet-gly2.

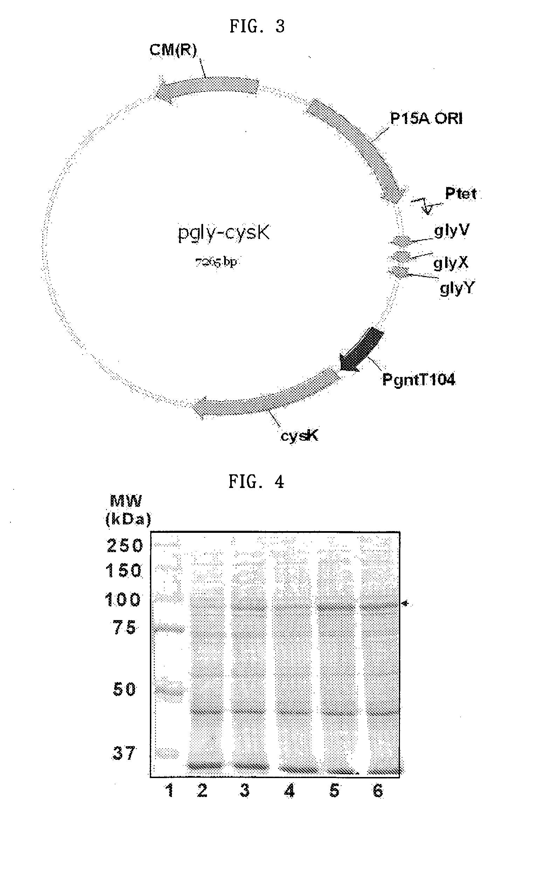

FIG. 3 is a gene map of the plasmid pgly-cysK.
FIG. 4 shows the results of observing the expression level of a silk protein consisting of 32 repeats by SDS-PAGE. In FIG. 4, lane 1: a marker showing protein standard molecular weight; lanes 2 and 4: the results of inducing the expression of a strain, transformed with the plasmids pSH32 and pACYC184, at OD₆₀₀ values of 0.2 and 0.4, respectively; and lanes 3, 5 and 6: the results of inducing the expression of a strain, transformed with the plasmids pSH32 and pTet-glyVXY, at OD₆₀₀ values of 0.2, 0.4 and 0.6, respectively.

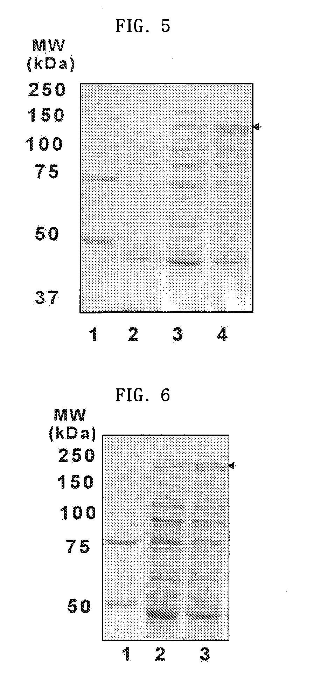

FIG. 5 shows the results of observing the expression level of a silk protein consisting of 48 repeats by SDS-PAGE. In FIG. 5, lane 1: a marker showing protein standard molecular weight; lane 2: the results of inducing the expression of a strain, transformed with the plasmids pET30a and pACYC184, at an OD₆₀₀ of 0.4; lane 3: the results of inducing the expression of a strain, transformed with the plasmids pSH48 and pACYC184, at an OD₆₀₀ of 0.4; and lane 4: the results of inducing the expression of a strain, transformed with the plasmids pSH48 and pTet-glyVXY, at an OD₆₀₀ of 0.4.
FIG. 6 shows the results of observing the expression level of a silk protein consisting of 64 repeats. In FIG. 6, lane 1: a marker showing protein standard molecular weight; lane 2: the results of inducing the expression of a strain, transformed with the plasmids pSH64 and pACYC184, at an OD₆₀₀ of 0.4; and lane 3: the results of inducing the expression of a strain, transformed with the plasmids pSH64 and ptet-gly2, at an OD₆₀₀ of 0.4.

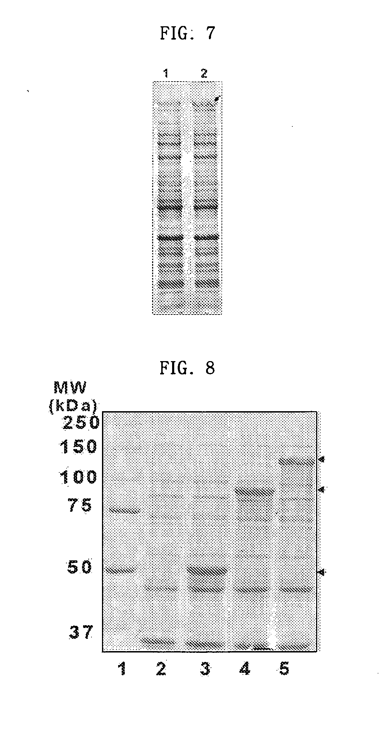

FIG. 7 shows the results of observing the expression of a new silk protein derived from Black Widow (*Latrodectus hesperus*) by SDS-PAGE. In FIG. 7, lane 1: the results of inducing the expression of a strain, transformed with the plasmids pBWA64 and pACYC184, at an OD₆₀₀ of 0.4; and lane 2: the results of inducing the expression of a strain, transformed with the plasmids pBWA64 and ptet-glyVXY, at an OD₆₀₀ of 0.4.
FIG. 8 shows the results of observing the expression level of a silk protein consisting of 48 repeats by SDS-PAGE. In FIG. 8, lane 1: a marker showing protein standard molecular weight; lane 2: the results of inducing the expression of a strain, transformed with the plasmids pET30a and pgly-cysK, at an OD₆₀₀ of 0.4; lane 3: the results of inducing the expression of a strain, transformed with the plasmids pSH16a and pgly-cysK, at an OD₆₀₀ of 0.4; lane 4: the results of inducing the expression of a strain, transformed with the plasmids pSH32 and pgly-cysK, at an OD₆₀₀ of 0.4; and lane 5: the results of a strain, transformed with the plasmids pSH48 and pgly-cysK, at an OD₆₀₀ of 0.4.

### BEST MODE FOR CARRYING OUT THE INVENTION

The present invention is directed to a method of increasing the expression of a target protein being silk protein having a high content of a specific amino acid. Also, the present invention is directed to a method of increasing the expression of the target protein by co-expression of a gene encoding the target protein acid with a nucleotide sequence encoding the tRNA of the specific amino acid being selected from the group consisting of glycine, alanine and serine using a recombinant microorganism transformed with both a gene encoding the target protein having a specific amino acid content of 10% or more and the nucleotide sequence encoding the tRNA of the specific amino acid.

In one Example of the present invention, a recombinant *E. coli* strain transformed with both a gene encoding silk protein resulting from the modification of a dragline silk protein obtained from *Nephila clavipes* and a nucleotide sequence encoding the glycine tRNA was constructed and cultured. As a result, it was found that a silk protein having a high glycine content (41%), a high alanine content (18%) and a high serine content (6.3%) was overexpressed. In addition, the silk protein is a repetitive protein consisting of repeats of a specific amino acid sequence (SGRGGLGGTGAGMAAAAAMGGAGQGGYGGLGSQG), and it was found that the expression of the silk protein consisting of each of 32, 48 and 64 repeats was significantly increased. In addition, it was found that, when the *cysK* gene known to stimulate the expression of a serine-rich protein was co-expressed with a nucleotide sequence encoding the glycine tRNA, the expression of the silk protein was further increased.

From the results in which the production of the silk protein having a high glycine content of 10% or more was increased due to the overexpression of the glycine tRNA, it will be obvious to a person of ordinary skill in the art that the overexpression of tRNA of a specific amino acid is useful for the production of not only a silk protein having a high glycine content, but also other proteins having a high content of a specific amino acid (10% or more).

Repetitive proteins having a high content of a specific amino acid include, in addition to the silk protein, elastin (GVGVP, VPGG, APGVGV), byssus (GPGGG), flagelliform silk (GPGGx), dragline silk (GPGQQ, GPGGY, GGYGPGS), collagen (GAPGAPGSQGAPGLQ, GAPGTPGPQGLPGSP), keratin (AKLKLAEAKLELA), sericin (SSTGSSSNTDSNSNSVGSSTSGGSSTYGYSSNSRDGSV) and synthetic repetitive protein. When the concept of the present invention is applied to byssus, the expression of byssus consisting of repeats of GPGGG can be increased by co-expression with the glycine or proline tRNA.

As a result, in the cases of not only the above-illustrated repetitive proteins, but also proteins having a high content of a specific amino acid (10% or more), the expression levels of the proteins can be increased by co-expression with tRNA of a specific amino acid.

In addition, the present invention illustrates only a recombinant microorganism transformed with both a recombinant vector containing a gene encoding the target protein and a recombinant vector containing a nucleotide sequence encoding tRNA of the specific amino acid, but a recombinant microorganism may also be obtained either by transforming a host microorganism with a recombinant vector containing both the target protein-encoding gene and tRNA of a specific amino acid or by inserting the target protein-encoding gene into the chromosome of a host microorganism and then introducing a recombinant vector, which contains a nucleotide sequence encoding tRNA of a specific amino acid, into the host microorganism. In addition, a recombinant microorganism may also be obtained by inserting both the target protein-encoding gene and the nucleotide sequence encoding tRNA of the specific amino acid into the chromosome of a host microorganism.

As used herein, the term "vector" means a DNA construct containing a DNA sequence operably linked to a suitable control sequence capable of effecting the expression of the DNA in a suitable host. The vector may be a plasmid, a phage particle, or simply a potential genomic insert. Once incorporated into a suitable host, the vector may replicate and function independently of the host genome, or may in some instances, integrate into the genome itself. In the present specification, "plasmid" and "vector" are sometimes used interchangeably, as the plasmid is the most commonly used form of vector. For the purpose of the present invention, the plasmid vector is preferably used. A typical plasmid vector which can be used for this purpose contains: (a) a replication origin by which replication occurs efficiently such that several hundred plasmid vectors per host cell are created; (b) an antibiotic-resistant gene by which host cells transformed with the plasmid vector can be selected; and (c) restriction enzyme cutting sites into which foreign DNA fragments can be inserted. Even if suitable restriction enzyme cutting sites are not present in the vector, the use of a conventional synthetic oligonucleotide adaptor or linker enables the easy ligation between the vector and the foreign DNA fragments. After ligation, the vector should be transformed into suitable host cells. The transformation can be easily achieved by the calcium chloride method or electroporation (Neumann, et al., EMBO J., 1:841, 1982).

As the vector which is used for the overexpression of a gene according to the present invention, an expression vector known in the art may be used.

A nucleotide sequence is operably linked when it is arranged in a functional relationship with another nucleic acid sequence. The nucleotide sequence may be a gene and a control sequence(s) linked to be capable of expressing the gene when it binds to a control sequence(s) (e.g., transcription-activating protein). For example, DNA for a pre-sequence or a secretory leader is operably linked to DNA encoding polypeptide when expressed as pre-protein participating in secretion of polypeptide; a promoter or an enhancer is operably linked to a coding sequence when affecting the transcription of the sequence; and a ribosome binding site is operably linked to a coding sequence when affecting the transcription of the sequence, or to a coding sequence when arranged to facilitate translation. Generally, the term "operably linked" means that the DNA linked sequences are contiguous, and in the case of the secretory leader, are contiguous and present in a reading frame. However, an enhancer is not necessarily contiguous. The linkage between these sequences is performed by ligation at a convenient restriction enzyme site. However, when the site does not exist, a synthetic oligonucleotide adaptor or a linker is used according to a conventional method.

As is well known in the art, in order to increase the expression level of a transfected gene in a host cell, a corresponding gene should be operably linked to transcription and translation expression control sequences which are operated in a selected expression host. Preferably, the expression control sequences and the corresponding gene are included in one recombinant vector together with a bacterial selection marker and a replication origin. When an expression host is a eukaryotic cell, an recombinant vector should further include an expression marker which is useful in a eukaryotic expression host.

The transformed cell constitutes another aspect of the present invention by the aforementioned recombinant vector. As used herein, the term "transformation" means that DNA can be replicated as a factor outside of chromosome or by means of completion of the entire chromosome by introducing DNA into a host.

Of course, it should be understood that all vectors do not equally function to express DNA sequences according to the present invention. Similarly, all hosts do not equally function with respect to the same expression system. However, one skilled in the art may appropriately select from among various vectors, expression control sequences, and hosts without either departing from the scope of the present invention or bearing excessive experimental burden. For example, a vector must be selected considering a host, because the vector must be replicated in the host. Specifically, the copy number of the vector, the ability of regulating the copy number and the expression of other protein encoded by the corresponding vector (e.g., the expression of an antibiotic marker) should also be considered.

Also, the present invention illustrates only *E. coli* as a host microorganism, but a person skilled in the art will appreciate that the host microorganism is not limited to *E*. *coli.* For example, other kinds of bacteria, yeasts, or fungi may also be used.

### EXAMPLES

Hereinafter, the present invention will be described in further detail with reference to examples.

### Example 1: Construction of recombinant plasmid pTet-glyVXY

All procedures for genetic manipulation were carried out according to standard methods (Sambrook et al., Molecular cloning: a laboratory manual, 2nd Ed., Cold Spring Harbor Laboratory Press, Cold Spring Harbor, New York, 1989). In order to obtain a *gly*VWX gene encoding the glycine tRNA, PCR was performed using a chromosome, isolated from an *E. coli* W3110 strain (derived from *E*. *coli* K-12, λ⁻, F⁻, prototrophic), as a template with primers of SEQ ID NO: 1 and SEQ ID NO: 2.
SEQ ID NO: 1: 5'-GCTCGATATCTAACGACGCAGAAATGCGAAA-3'
SEQ ID NO: 2: 5'-CATTGGATCCTAAGATTACAGCCTGAGGCTGTG-3'

The PCR reaction was performed using *Pfu* polymerase (SolGent, Korea) under the following conditions: initial denaturation at 95 °C for 4 min; then 10 cycles of denaturation at 95 °C for 20 sec, annealing at 51 °C for 30 sec and extension at 72 °C for 60 sec; and then 19 cycles of denaturation at 95 °C for 20 sec, annealing at 60 °C for 30 sec and extension at 72 °C for 60 sec; followed by final extension at 72 °C for 5 min.

The DNA obtained by the PCR reaction was electrophoresed on agarose gel electrophoresis to obtain a purified 479-bp PCR product. The PCR product was digested with the restriction enzymes *Bam*HI and *Eco*RV (New England Biolabs, USA), and in order to use the promoter of a tetracycline-resistant gene (*tet*) which can be continuously expressed, plasmid pACYC184 (New England Biolabs, USA) was also digested with the same restriction enzymes. The digested PCR product and plasmid were ligated to each other using T4 DNA ligase (Roche, Germany) and transformed into *E. coli* Top10 (F⁻*mcr*A Δ(*mrr*-*hsd*RMS-*mcr*BC) ¢0*lac*ZΔM15 Δ*lacX*74 recA1 araD139 Δ*(ara-leu)7697 gal*U *gal*K, *rps*L (Str^{R}) *end*A1 *nup*G). The transformed strain was selected on LB agar solid medium (10 g/L trypton, 5 g/L yeast extract, 5 g/L NaCl, and 15 g/L agar) containing 34 mg/L chloramphenicol, thus constructing the recombinant plasmid pTet-glyVXY (FIG. 1). The constructed recombinant plasmid was confirmed by digestion with restriction enzymes and nucleotide sequence analysis.

### Example 2: Construction of recombinant plasmid pTet-gly2

All procedures for gene manipulation were carried out according to standard methods (Sambrook et al., Molecular cloning: a laboratory manual, 2nd Ed., Cold Spring Harbor Laboratory Press, Cold Spring Harbor, New York, 1989). To further overexpress a *gly*VXY gene encoding the glycine tRNA, PCR was performed using pTet-glyVXY as a template with primers of SEQ ID NO: 3 and SEQ ID NO: 4.
SEQ ID NO: 3: 5'-GGCTCGCATGCTCATGTTTGACAGCTTATCATCGA-3'
SEQ ID NO: 4:
   5'-ATTGTCGACTGCTGCAGTAAGATTACAGCCTGAGGCTGTG-3'

The PCR reaction was performed using *Pfu* polymerase (SolGent, Korea) under the following conditions: initial denaturation at 95 °C for 3 min; then 10 cycles of denaturation at 95 °C for 20 sec, annealing at 52 °C for 30 sec and extension at 72 °C for 50 sec; and then 19 cycles of denaturation at 95 °C for 20 sec, annealing at 62 °C for 30 sec and extension at 72 °C for 50 sec; followed by final extension at 72 °C for 5 min.

The DNA obtained by the PCR reaction was electrophoresed on agarose gel to obtain a purified 674-bp PCR product. The 647-bp PCR product and the plasmid pTet-glyVXY were digested with the restriction enzymes *Sph*I and *Sal*I (New England Biolabs, USA) and ligated to each other by T4 DNA ligase (Roche, Germany), and then transformed into *E*. *coli* Top1O The transformed strain was selected on LB agar solid medium (10 g/L tryptone, 5 g/L yeast extract, 5 g/L NaCl, and 15 g/L agar) containing 34 mg/L chloramphenicol, thus constructing the recombinant plasmid pTet-gly2 (FIG. 2). The constructed recombinant plasmid was confirmed by digestion with restriction enzymes and nucleotide sequence analysis.

### Example 3: Construction of recombinant plasmid pgly-cysK

All procedures for gene manipulation were carried out according to standard methods (Sambrook et al., Molecular cloning: a laboratory manual, 2nd Ed., Cold Spring Harbor Laboratory Press, Cold Spring Harbor, New York, 1989). In order to overexpress the glycine tRNA gene together with the cysK gene encoding cycteine synthase A, plasmid pter-gly2 was digested with the restriction enzyme *Sal*I (New England Biolabs, USA) and treated with Klenow enzyme to make blunt ends, and then digested with the restriction enzyme *Cla*I.

Then, the digested plasmid was ligated with plasmid pAC104CysK (Han et al., Appl. Environ. Microbiol., 69(10) :5772, 2003) digested with the restriction enzymes *Cla*I and *Eco*RV, thus constructing the recombinant plasmid pgly-cysK (FIG. 3). The constructed recombinant plasmid was confirmed by digestion with restriction.

### Example 4: Construction of recombinant plasmids pSH32, pSH48, pSH64 and pBWA64

All procedures for gene manipulation were carried out according to standard methods (Sambrook et al., Molecular cloning: a laboratory manual, 2nd Ed., Cold Spring Harbor Laboratory Press, Cold Spring Harbor, New York, 1989). In order to construct the recombinant plasmid pSH32, the plasmid pSH16a (Lee et al., Theories and Applications of Chem. Eng., 8(2):3969, 2002) was digested with the restriction enzymes *Spe*I and *Nhe*I (New England Biolabs, USA) to obtain a 1.7-kb fragment, which was then ligated with the plasmid pSH16a digested with the restriction enzyme *Spe*I, thus obtaining the recombinant plasmid pSH32. The direction of the ligated insert was determined by digestion with the restriction enzyme *Spe*I and *NheI.* In the same manner, the plasmid pSH16a was digested with the restriction enzymes *Spe*I and *Nhe*I to obtain a 1.7-kb fragment, which was then ligated with the plasmid pSH32 digested with the restriction enzyme *Spe*I, thus obtaining the recombinant plasmid pSH48. Also, the plasmid pSH32 was digested with the restriction enzymes *Spe*I and *Nhe*I to obtain a 3.4-kb fragment, which was then ligated with the plasmid pSH32 digested with the restriction enzyme *Spe*I, thus obtaining the recombinant plasmid pSH64. The direction of each of the ligated inserts was determined by digestion with the restriction enzymes *Spe*I and *Nhe*I*.*

Also, in order to construct the recombinant vector pBWA64 for synthesizing a new silk protein derived from Black Widow (*L. hesperus*), overlapping PCR was performed using primers of SEQ ID NOS: 5 and 6, and the PCR product was digested with NdeI and XhoI and inserted into pET30a, thus constructing the plasmid pBWA1. Then, a fragment obtained by digesting the pBWA1 vector with SpeI and XmaI was ligated with a fragment obtained by digesting the pBWA1 vector with NheI and XmaI, thus constructing pBWA2. pBW2 was digested with a combination of the same enzymes as described above, and ligated, thus constructing pBWA4. According to this method, pBWA8, pBWA16, pBWA32, and finally pBWA64 were constructed.
SEQ ID NO: 5: SEQ ID NO: 6:

### Example 5: Expression of silk protein consisting of 32 repeats by co-overexpression of glycine tRNA

In order to confirm the effect of the co-overpexpression of the glycine tRNA gene on the production of a silk protein, the plasmids pTet-glyVXY and pSH32, obtained in Examples 1 and 4, were introduced into *E coli* BL21 (DE3) (*F-ompT hsdS*B(*rB- mB*-) *gal dcm* (DE3), a prophage carrying the T7 RNA polymerase gene) (New England Biolabs, USA). As a control group, an *E coli* BL21 (DE3) strain transformed with the plasmids pACYC184 and pSH32 was used.

The transformed strains were inoculated into LB liquid medium (10 g/L tryptone, 5 g/L yeast extract, and 5 g/L NaCl) containing 34 mg/L chloramphenicol and 25 mg/L kanamycin and were cultured with continuous shaking at 180 rpm at 30 °C. When the optical density (O.D.) measured with a spectrophotometer at a wavelength of 600 nm after inoculation of 1% of each strain reached 0.2, 0.4 and 0.6, 1 mM IPTG was added to each strain to induce the expression of the silk protein gene. 5 hours after induction of the expression of the silk protein gene, the cultures were harvested. For recombinant protein analysis, each of the harvested cultures was centrifuged at 4 °C at 10,000 g for 10 minutes to obtain cell pellets which were then dissolved in TE buffer and 5x Laemmli sample buffer. The same amount (0.024 mg) of samples were taken from the cultures using 10% SDS-PAGE and stained with Coomassie brilliant blue R250 (Bio-Rad, USA), followed by quantification with GS-710 Calibrated Imaging Densitometer (Bio-Rad, USA) (FIG. 4).

As a result, it could be seen that the expression of the silk protein consisting of 32 repeats was increased by about 50% compared to the control group due to the overexpression of the glycine tRNA regardless of the time point at which the expression of the silk protein was induced.

### Example 6: Expression of silk protein consisting of 48 repeats by co-overexpression of glycine tRNA

In order to confirm the effect of the co-overexpression of the glycine tRNA gene on the production of silk protein, the plasmids pTet-glyVXY and pSH48 obtained in Examples 1 and 4 were introduced into *E*. *coli* BL21 (DE3) (*F- ompT hsdSB*(*rB-mB*-) *gal dcm* (DE3), a prophage carrying the T7 RNA polymerase gene) (New England Biolabs, USA). As a control group, an E coli strain BL21 (DE3) strain transformed with the plasmids pACYC184 and pET30a or with the plasmids pACYC184 and pSH48 was used.

The transformed strains were cultured under the same conditions as in Example 5, and the expression of the silk protein in the transformed strains was observed by SDS-PAGE (FIG. 5). As a result, it was found that the silk protein consisting of 48 repeats was increased by about three times compared to the control group due to the overexpression of the glycine tRNA.

### Example 7: Expression of silk protein consisting of 64 repeats by co-overexpression of glycine tRNA

In order to confirm the effect of the co-overexpression of the glycine tRNA gene on the production of silk protein, the plasmids pTet-glyVXY and pSH64 obtained in Examples 1 and 4 were introduced into *E*. *coli* BL21 (DE3) (*F- ompT hsd*SB(*rB-mB*-) *gal dcm* (DE3), a prophage carrying the T7 RNA polymerase gene) (New England Biolabs, USA). As a control group, an *E*. *coli* BL21 (DE3) strain transformed with the plasmids pACYC184 and pSH64 was used.

The transformed strains were cultured under the same conditions as in Example 5, and the expression of the silk protein in the strains was analyzed by SDS-PAGE (FIG. 6). As a result, it was shown that the expression of the silk protein consisting of 64 repeats was increased by about 5 times compared to the control group due to the overexpression of the glycine tRNA.

### Example 8: Expression of new synthetic silk protein derived from Black Widow (Latrodectus hesperus) by co-overexpression of glycine tRNA

In order to confirm the effect of the co-overexpression of the tRNA gene on the production of silk protein, the plasmids pTet-glyVXY and pBWA64 obtained in Examples 1 and 4 were introduced into *E*. *coli* BL21 (DE3) (*F- ompT hsdSB*(*rB-mB*-) *gal dcm* (DE3), a prophage carrying the T7 RNA polymerase gene) (New England Biolabs, USA). As a control group, an *E*. *coli* BL21 (DE3) strain transformed with the plasmids pACYC184 and pBWA64 was used.

The transformed strains were cultured under the same conditions as in Example 5, and the expression of silk protein in the transformed strains was observed by SDS-PAGE (FIG. 7). As a result, it was shown that a new silk protein derived from Black Widow (*L. hesperus*) was increased by about three times compared to the control group due to the overexpression of the glycine tRNA.

### Example 9: Expression of silk protein consisting of several repeats by co-overexpression of glycine tRNA and cysK gene

It was demonstrated that the expression of a serine-rich protein is increased by co-expression with the cysK gene (KR 10-0489500). The silk protein used in this Example has a high glycine content (41%) and a high serine content (6.3%) and requires serine as a direct precursor for glycine biosynthesis. In order to confirm the effect of co-expression of the glycine tRNA gene and the *cysK* gene, four transformed strains were constructed by *E. coli* BL21 (DE3) (*F- ompT hsdSB* (*rB- mB*-) *gal dcm* (DE3), a prophage carrying the T7 RNA polymerase gene, New England Biolabs, USA) with two plasmids, the plasmid pgly-cysK obtained in Example 3 and pET30a, pSH16a, or pSH32 or pSH64 obtained in Example 4.

The transformed strains were cultured under the same conditions as in Example 5, and the expression of silk protein in the transformed strains was observed by SDS-PAGE (FIG. 8). As a result, it was shown that, due to the co-overexpression of the glycine tRNA and the cysK gene, the expression of a silk protein consisting of several repeats was significantly increased, and the growth of the silk protein was also promoted.

### INDUSTRIAL APPLICABILITY

As described above in detail, according to the present invention, the expression of silk protein having a high content of a specific amino acid being selected from the group consisting of glycine, alanine and serine can be remarkably increased by co-expression with the tRNA of the specific amino acid. Thus, the present invention is useful for increasing the productivity of a protein having a high content of a specific amino acid, such as a repetitive protein.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof. All these simple variations and modifications of the embodiment can be made by a person of ordinary skill in the art, and it is intended to cover in the appended claims all such modifications that fall within the scope of the invention.

## Claims

1. A recombinant microorganism transformed with both a gene encoding a target protein having a specific amino acid content of 10% or more and a nucleotide sequence encoding the tRNA of the specific amino acid wherein the target protein is silk protein and the specific amino acid is selected from the group consisting of glycine, alanine and serine.

2. The recombinant microorganism according to claim 1, wherein the target protein is a repetitive protein having repeats of specific oligopeptides.

3. The recombinant microorganism according to claim 1, wherein the recombinant microorganism is *E.coli.*

4. A recombinant vector containing both a gene encoding a target protein having a specific amino acid content of 10% or more and a nucleotide sequence encoding the tRNA of the specific amino acid, wherein the target protein is silk protein and the specific amino acid is selected from the group consisting of glycine, alanine and serine.

5. The recombinant vector according to claim 4, wherein the target protein is a repetitive protein having repeats of specific oligopeptides.

6. The recombinant vector according to claim 4, wherein the recombinant vector comprises T7 promoter.

7. A recombinant microorganism transformed with the recombinant vector of claim 4.

8. The recombinant microorganism according to claim 7, wherein the recombinant microorganism is *E.coli.*

9. A method of preparing a protein having a specific amino acid content of 10% or more, **characterized in that** the method comprises culturing said recombinant microorganism according to claim 1 to express a target protein having a specific amino acid content of 10% or more, and collecting the expressed target protein, wherein the target protein is silk protein and the specific amino acid is selected from the group consisting of glycine, alanine and serine.

10. The method according to claim 9, wherein the target protein is a repetitive protein having repeats of specific oligopeptides.

## Patentansprüche

1. Rekombinanter Mikroorganismus, transformiert mit sowohl einem Gen, welches ein Zielprotein mit einem Anteil einer spezifischen Aminosäure von 10% oder mehr codiert, als auch mit einer Nukleotidsequenz, welche die tRNA der spezifischen Aminosäure codiert, wobei das Zielprotein Seidenprotein ist und die spezifische Aminosäure ausgewählt ist aus der Gruppe bestehend aus Glycin, Alanin und Serin.

2. Rekombinanter Mikroorganismus gemäß Anspruch 1, wobei das Zielprotein ein Wiederholungsprotein mit Wiederholungseinheiten spezifischer Oligopeptide ist.

3. Rekombinanter Mikroorganismus gemäß Anspruch 1, wobei der rekombinante Mikroorganismus *E*. *coli* ist.

4. Rekombinanter Vektor, enthaltend sowohl ein Gen, welches ein Zielprotein mit einem Anteil einer spezifischen Aminosäure von 10% oder mehr codiert, als auch eine Nukleotidsequenz, welche die tRNA der spezifischen Aminosäure codiert, wobei das Zielprotein Seidenprotein ist und die spezifische Aminosäure ausgewählt ist aus der Gruppe bestehend aus Glycin, Alanin und Serin.

5. Rekombinanter Vektor gemäß Anspruch 4, wobei das Zielprotein ein Wiederholungsprotein mit Wiederholungseinheiten von spezifischen Oligopeptiden ist.

6. Rekombinanter Vektor gemäß Anspruch 4, wobei der rekombinante Vektor einen T7-Promotor umfasst.

7. Rekombinanter Mikroorganismus, welcher mit dem rekombinanten Vektor gemäß Anspruch 4 transformiert ist.

8. Rekombinanter Mikroorganismus gemäß Anspruch 7, wobei der rekombinante Mikroorganismus *E*. *coli* ist.

9. Verfahren zur Synthetisierung eines Proteins mit einem Anteil einer spezifischen Aminosäure von 10% oder mehr, **dadurch gekennzeichnet , dass** das Verfahren Kultivieren des rekombinanten Mikroorganismus gemäß Anspruch 1, um ein Zielprotein zu exprimieren, das einen Anteil einer spezifischen Aminosäure von 10% oder mehr besitzt, und Sammeln des exprimierten Zielproteins umfasst, wobei das Zielprotein Seidenprotein ist und die spezifische Aminosäure ausgewählt wird aus der Gruppe bestehend aus Glycin, Alanin und Serin.

10. Verfahren gemäß Anspruch 9, wobei das Zielprotein ein Wiederholungsprotein mit Wiederholungseinheiten von spezifischen Oligopeptiden ist.

## Revendications

1. Micro-organisme recombinant transformé avec à la fois un gène codant pour une protéine cible ayant une teneur en un acide aminé spécifique de 10 % et plus et une séquence nucléotidique codant pour l'ARNt de l'acide aminé spécifique, où la protéine cible est une protéine de soie et l'acide aminé spécifique est choisi dans le groupe constitué de glycine, alanine et sérine.

2. Micro-organisme recombinant selon la revendication 1, où la protéine cible est une protéine répétitive comportant des répétitions d'oligopeptides spécifiques.

3. Micro-organisme recombinant selon la revendication 1, le micro-organisme recombinant étant *E*. *coli.*

4. Vecteur recombinant contenant à la fois un gène codant pour une protéine cible ayant une teneur en un acide aminé spécifique de 10 % ou plus et une séquence nucléotidique codant pour l'ARNt de l'acide aminé spécifique, où la protéine cible est une protéine de soie et l'acide aminé spécifique est choisi dans le groupe constitué de glycine, alanine et sérine.

5. Vecteur recombinant selon la revendication 4, où la protéine cible est une protéine répétitive comportant des répétitions d'oligopeptides spécifiques.

6. Vecteur recombinant selon la revendication 4, où le vecteur recombinant comprend le promoteur T7.

7. Micro-organisme recombinant transformé avec le vecteur recombinant selon la revendication 4.

8. Micro-organisme recombinant selon la revendication 7, le micro-organisme recombinant étant *E*. *coli.*

9. Procédé de préparation d'une protéine ayant une teneur en un acide aminé spécifique de 10 % ou plus, **caractérisé en ce que** le procédé comprend la culture dudit micro-organisme recombinant selon la revendication 1 pour exprimer une protéine cible ayant une teneur en un acide aminé spécifique de 10 % ou plus, et le recueil de la protéine cible exprimée, où la protéine cible est une protéine de soie et l'acide aminé spécifique est choisi dans le groupe constitué de glycine, alanine et sérine.

10. Procédé selon la revendication 9, dans lequel la protéine cible est une protéine répétitive comportant des répétitions d'oligopeptides spécifiques.
